Europäisches Patentamt

European Patent Office

Office européen des brevets

(19)

(11) EP 0 509 026 B1

(12) EUROPÄISCHE PATENTSCHRIFT

(45) Veröffentlichungstag und Bekanntmachung des Hinweises auf die Patenterteilung:
**18.03.1998 Patentblatt 1998/12**

(21) Anmeldenummer: **91902118.8**

(22) Anmeldetag: **19.12.1990**

(51) Int. Cl.$^6$: **A61K 49/00**, G01N 33/58

(86) Internationale Anmeldenummer:
**PCT/EP90/02257**

(87) Internationale Veröffentlichungsnummer:
**WO 91/09628 (11.07.1991 Gazette 1991/15)**

(54) **VERWENDUNG EINES ANTIKOAGULANT ALS DIAGNOSTIKUM**

USE OF AN ANTICOAGULANT AS A DIAGNOSTIC AGENT

UTILISATION D'UN ANTICOAGULANT COMME MOYEN DE DIAGNOSTIC

(84) Benannte Vertragsstaaten:
**AT BE CH DE DK ES FR GB GR IT LI LU NL SE**

(30) Priorität: **27.12.1989 DE 3942988**

(43) Veröffentlichungstag der Anmeldung:
**21.10.1992 Patentblatt 1992/43**

(60) Teilanmeldung:
**97113801.1 / 0 806 670**

(73) Patentinhaber:
**BOEHRINGER INGELHEIM INTERNATIONAL GmbH**
**55218 Ingelheim am Rhein (DE)**

(72) Erfinder:
**REUTELINGSPERGER, Christiaan**
**NL-6211 JK Maastricht (NL)**

(56) Entgegenhaltungen:
**EP-A- 0 063 002**      **EP-A- 0 293 567**
**DE-A- 3 810 331**      **US-A- 4 455 290**
**US-A- 4 820 505**

- **THE JOURNAL OF BIOLOGICAL CHEMISTRY, Band. 264, Nr. 14, Mai 1989; Jonathan F. Tait et al: "Phospholipid Binding Properties of Human Placental Anticoagulant Protein-I, a Member of the Lipocortin Family ", p. 7945, column 1, line 24-38**

EP 0 509 026 B1

**Beschreibung**

Gegenstand der vorliegenden Erfindung sind Verwendung von Mittel, insbesondere Annexine, die mit einer detektierbaren Substanz markiert sind als Diagnos.

Blut besteht aus speziellen ungebundenen Zellen, die in einem Plasma-Medium dispergiert sind. Der Zellinhalt ist von dem umgebenden Plasma durch sogenannte Plasma-Membranen getrennt. Diese Membranen sind aus Phospholipiden in Form einer Doppelschicht aufgebaut und aus assoziierten Proteinen, die teilweise diese Doppelschicht penetrieren bzw. aus ihnen herausragen.

Die verschiedenen Phospholipide sind nicht wahllos über die äußere und innere Hülle der Doppelschicht verteilt, sondern werden durch die Zelle in einer asymmetrischen Konfiguration gehalten (7,8). Während Phosphatidylcholin (PC) und Sphingomyelin (SPH) die dominierenden Spezies der äußeren Hülle sind, sind Phosphatidylserin (PS), Phosphatidyiethanolamin (PE) und Phosphatidylinositol (PI) vorwiegend in der inneren Hülle lokalisiert, dem Cytosol zugewandt. Dieser energieverbrauchende Asymmetriezustand ist von außerordentlicher physiologischer Bedeutung. PC und SPH sind die am stärksten inerten Spezies der Phospholipidfamilie und weisen in krassem Gegensatz zu den anderen Speziesein äußerst neutrales Verhalten gegenüber den Plasmakomponenten auf. Diese Reaktionsträgheit der äußeren Hülle gegenüber den Plasmaproteinen ist absolute Voraussetzung, um den flüssigen Zustand des Blutes zu gewährleisten. Spezielle Plasmaproteine, die zur Blutgerinnungskaskade gehören, die sogenannten Koagulationsfaktoren, können nämlich den flüssigen Blutzustand in einen festen Zustand überführen, wenn sie aktiviert werden (9). Aktiviert werden können diese Koagulationsfaktoren durch Phospholipide wie Phosphatidylserin.

In manchen Fällen, z.B. nach Verletzung eines Blutgefäßes, ist es erforderlich, ja gar lebensnotwendig, daß die Koagulationsfaktoren aktiviert werden. In einer solchen Situation kann eine spezielle Blutzelle, das Plättchen, seine Membran-Asymmetrie durch Aktivierungsmechanismen, die Phosphatidylserin zur äußeren Hülle transportieren, aufgeben, wo es die Aktivierung der Koagulationsfaktoren unterstützt (10).

Diese planmäßige Änderung der Phospholipidzusammensetzung der äußeren Hülle der Plättchen Plasma-Membran, hat große physiologische Bedeutung für die Hämostase, wie beispielsweise durch das Scott-Syndrom (11) angedeutet.

Zur Physiologie gehört aber auch die Pathologie; so kann die Homöostase des Blutes in einigen Fällen auch in einen pathologischen Zustand abgleiten, wie es bei arteriellen, coronaren und venösen Thrombosen der Fall ist.

Diese hämostatischen Störungen sind meistens idiopathisch und machen es den Ärzten unmöglich, ihr Auftreten vorherzusagen und prophylaktische Therapien zu entwickeln.

Aufgabe der vorliegenden Erfindung war es, Hilfsmittel bereitzustellen, die hämostatische Störungen frühzeitig erkennen helfen.

Im Gegensatz zur Entwicklung der Symptome läuft die Entwicklung der Störungen in den meisten Fällen langsam ab. Während dieser Phase symptomloser Progression, dem sogenannten prothrombotischen Zustand, läuft lokal eine kontinuierliche Aktivierung des Koagulationssystems ab. Verbunden mit dieser lokalen Aktivierung ist in der Peripherie das Auftreten von Plättchen, die sich in einem frühen Zustand der Aktivierung befinden. Diese Plättchen haben bereits damit begonnen, die Phospholipidzusammensetzung ihrer äußeren Plasmamembranhülle zu ändern. Phosphatidylserin ist in der äußeren Hülle vorhanden. Hilfsmittel, die diesen sogenannten prothrombotischen Zustand diagnostizieren, wären von größter kinischer Bedeutung.

Gegenstand der vorliegenden Erfindung sind auch die Verwendung von Hilfsmittel, die spezifisch Phosphatidylserin von Phosphatidylcholin unterscheiden können.

Neben dem Auftreten von schwach aktivierten Plättchen in der Peripherie, werden sich vollständig aktivierte Plättchen dort anheften, an der sich die Gefäßwand in pathologischer Kondition befindet. Diese Stelle ist als Auslöser für die Aktivierung des hämostatischen Systems anzusehen. Die Lokalisierung dieser pathologischen Stelle sowie die des sich hier bildenden Thrombus wäre von größtem therapeutischen Interesse.

Gegenstand der vorliegenden Erfindung sind daher auch die Verwendung von Mittel mit denen der Ausgangspunkt der Aktivierung des hämostatischen Systems und/oder ein Thrombus zu lokalisieren ist.

Der Blutgerinnungsmechanismus stellt sich als eine Kaskade enzymatischer Reaktionen dar, an deren Ende die Bildung von Thrombin steht, das letztendlich Fibrinogen in Fibrin umwandelt. Verschiedene prokoagulante Reaktionen wie beispielsweise die Aktivierung von Prothrombin durch die Faktoren Xa und Va werden katalysiert durch Phospholipidoberflächen, an die die Koagulationsfaktoren binden.

Bei den Proteinen, die an Phospholipide binden und mit Phospholipidoberflächen abhängigen Prozessen interferieren, gibt es eine Familie, die in ihrer Bindung an Phospholipide $Ca^{2+}$ abhängig sind.

Zu dieser Familie, die auch Annexine genannt wird, gehört neben Lipocortin I, Calpactin I, Protein II, Lipocortin III, p67-Calelectrin auch das Vascular Antikoagulant Protein (VAC) und IBC, PAP, PAPI, PP4, Endonexin II und Lipocortin V.

Die gemeinsamen strukturellen Merkmale der Annexine sind wahrscheinlich die Grundlagen für ihre ähnlichen $Ca^{2+}$ und Phospholipidbindungseigenschaften. Obwohl diese generelle Eigenschaft für alle Annexine gilt, besteht eine klare Individualität hinsichtlich ihrer Affinität zu $Ca^{2+}$ und zu den verschiedenen Phospholipidarten.

Die physiologischen Funktionen der Annexine betreffen membranassoziierte Prozesse. Der grundlegende Mechanismus der gerinnungshemmenden Wirkung des VAC wurde als eine Hemmung der katalytischen Kapazität der Phospholipide durch die Bindung des VAC an ihre Oberfläche erkannt, wodurch die Bildung des gerinnungsfördernden Komplexes an ihrer Oberfläche verhindert wird.

Bindungsstudien haben gezeigt, daß sich VAC Calcium-abhängig mit prokoagulatorischen Phospholipiden reversibel assoziiert.

Auch andere bivalente Kationen aus der Reihe $Cd^{2+}$, $Zn^{2+}$, $Mn^{2+}$ und $Co^{2+}$ beeinflussen die Assoziation positiv, jedoch nicht in dem Maße wie $Ca^{2+}$.

Darüberhinaus wurde überraschenderweise gefunden, daß die VAC Adsorption an Phospholipide in Gegenwart von $Ca^{2+}$- und $Zn^{2+}$-Ionen außerordentlich positiv beeinflußt wird.

Überraschenderweise wurde festgestellt, daß VAC bei Plasma-Calziumkonzentrationen an Phosphatidylserin aber nicht an Phosphatidylcholin und Sphingomyelin bindet. VAC erkennt und bindet daher spezifisch periphere Plättchen mit PS in ihrer äußeren Membranhülle. Darüberhinaus erkennt VAC auch solche Stellen im vaskularen System spezifisch, die PS dem Blut aussetzen.

Diese Differenzierung bei den Phospholipiden macht VAC ebenso wie die anderen Annexine zu einem idealen Reagenz, um den sogenannten prothrombotischen Zustand, der sich wie oben geschildert darstellt, frühzeitig zu erkennen.

Durch die vorliegende Erfindung werden Mittel bereitgestellt, mit denen es überraschenderweise erstmals möglich ist, den prothrombotischen Zustand des vaskularen Systems zu erkennen. Ermöglicht wird diese Diagnose durch die Spezifität von Substanzen, von Mitteln, die in der Lage sind, den gegenüber dem Normalzustand veränderten, den prothrombotischen Zustand der Plättchen zu erkennen. Da sich dieser Zustand von dem Normalzustand der Plättchen dadurch unterscheidet, daß die äußere Hülle nur des prothrombotischen Plättchens Phosphatidylserin aufweist, ist zur Ausnutzung dieses erfindungsgemäßen Prinzips im Prinzip jedes Mittel geeignet, das Phosphatidylserin spezifisch von Phosphatidylcholin unterscheiden kann. Die erfindungsgemäß einsetzbaren Mittel sind durch ihre Spezifität zu Phosphatidylserin gekennzeichnet, die durch die in der Beschreibung angegebenen Bindungsversuche zu ermitteln ist.

Die Ausnutzung dieser Spezifität der erfindungsgemäßen Mittel macht es darüber hinaus auch möglich, den Ausgangspunkt für die Aktivierung des hämostatischen Systems und/oder den Thrombus zu lokalisieren.

Mit der vorliegenden Erfindung werden damit erstmals Hilfsmittel bereitgestellt, die es durch frühzeitige Diagnose eines möglicherweise sich entwickelnden gesundheitsgefährdenden Zustands ermöglichen, geeignete therapeutische Maßnahmen zu ergreifen.

Bevorzugte erfindungsgemäße Mittel sind antikoagulierende Polypeptide, sofern sie die notwendige Spezifität zum Phospholipid Phosphatidylserin aufweisen.

Besonders bevorzugt ist die Familie der Annexine, insbesondere das VAC.

Um die erfindungsgemäßen Mittel, insbesondere VAC bzw. die anderen Annexine als Diagnostikum einsetzen zu können, werden sie in an sich bekannter Weise markiert. Als geeignete Marker bietet sich beispielsweise die Markierung mit fluoreszierenden Gruppen, oder die radioaktive Markierung an. Als vorteilhaft einsetzbarer Fluoreszenzmarker ist Fluoresceinisothiocyanat zu nennen, als vorteilhaft einsetzbare Radioaktivmarker die Radioisotope der Halogene, insbesondere die des Iod, beispielsweise $^{131}$I oder $^{125}$I oder auch Blei, Quecksilber, Thallium, Technetium oder Indium ($^{203}$Pb, $^{198}$Hg, $^{201}$Tl, $^{99m}$Tc, $^{111}$In) an.

Fluoresceinisothiocyanat (Serva) kann in an sich bekannter Weise (13) zur Markierung von VAC eingesetzt werden.

Auch möglich ist die Markierung mit einem paramagnetischen Kontrastagenz, das in einem MRI System (magnetic resonance imaging) detektierbar ist. Verwendet werden können Gadolinium, Kobalt, Nickel, Mangan oder Eisenkomplexe mit denen Konjugate als diagnostische Agenzien bereitgestellt werden können, die in einem MRI System detektierbar sind. In solchen Systemen wird ein starkes Magnetfeld verwendet, um die nuklearen Spinvektoren der Atome im Organismus auszurichten. Anschließend wird das Feld gestört, wodurch die Nuklei in ihren Ausgangszustand zurückkehren. Dieser Vorgang wird beobachtet und festgehalten.

Das so mit einem detektierbaren Marker versehene antikoagulierende Polypeptid wird dann auf intraarteriellem oder intravenösem Weg verabreicht. Die applizierte Menge ist so zu bemessen, daß sie für die nachfolgende Messung, nach genügender Inkubationszeit, ausreicht.

Zum Nachweis eines prothrombotischen Zustands wird dem zu untersuchenden Blut extrakorporal das erfindungsgemäß markierte Polypeptid oder Mittel zugegeben, gegebenenfalls in Anwesenheit eines weiteren, nicht die Plasma-Calciumkonzentration vermindernden Antikoagulants wie beispielsweise Heparin, woraufhin dann die mit spezifischen Zelltypen assoziierte Markierung analysiert wird.

Das erfindungsgemäß markierte Polypeptid kann sowohl in blutisotonischer wäßriger Lösung als auch mit Hilfsstoffen zum erfindungsgemäßen Zweck eingesetzt werden. Als Hilfsstoffe können beispielsweise Arginin, Phosphatpuffer und physiologisch verträgliche Konservierungsstoffe eingesetzt werden. Weitere Stoffe sind dem Fachmann

EP 0 509 026 B1

bestens bekannt und können ebenfalls eingesetzt werden.

Zur Durchführung der radioaktiven Markierung verwendet man z.B. die bekannte Iodogen-Methode (12) oder die gebräuchliche Chloramin-T-Methode. Wegen seiner Halbwertszeit von 8 Tagen ist für die in vivo Diagnose $^{131}$I empfehlenswert. Das radioaktiv markierte Mittel wird in blutisotonischer wäßriger Lösung aufgenommen. Nach Sterilfiltration wird es injiziert. Die Ganzkörperszintigraphien werden mit einer Gammakamera z.B. für $^{131}$I 1, 2, 4 und 7 Tage nach der Injektion durchgeführt.

Neben den genuinen Formen der Annexine sind auch veränderte Formen für die erfindungsgemäße Verwendung einsetzbar.

Verwiesen sei insbesondere auf die in der EPA 0 293 567 beschriebenen Mutanten. Darüberhinaus sind auch die Fragmente oder chemisch modifizierte Derivate der Annexine verwendbar, die die Spezifität bezüglich der Phospholipide Phosphatidylserin/Phosphatidylcholin aufweisen und somit den prothrombotischen Zustand der beteiligten Plättchen erkennen können.

Gegenstand der vorliegenden Erfindung sind im Einzelnen:
Die Verwendung von

- Antikoagulierendes Polypeptid aus der Familie der Annexine, vorzugsweise VAC, das mit einem detektierbaren Marker versehen ist.

Die Verwendung von

- Antikoagulierendes Polypeptid, das als detektierbaren Marker die Fluoreszenzmarkierung vorzugsweise Fluoresceinisothiocyanat, ein Radioisotop eines Halogens, des Technetiums, Bleis, Quecksilbers, Thalliums oder des Indiums, besonders bevorzugt $^{131}$I oder $^{125}$I oder ein paramagnetisches Kontrastagenz enthält.

Die Verwendung von

- Antikoagulierendes Polypeptid wie oben beschrieben zur Unterscheidung von Phophatidylserin und Phosphatidylcholin.

Die Verwendung von

- Antikoagulierendes Polypeptid wie oben beschrieben als Diagnostikum.

- Verfahren zur Feststellung des Ausgangspunktes für die Aktivierung des hämostatischen Systems, bei dem

    a) ein mit einem detektierbaren Marker versehenes antikoagulierendes Polypeptid oder Mittels aus der Familie der Annexine, vorzugsweise VAC in das System eingebracht wird, vorzugsweise intraarteriell oder intravenös und

    b) nach einer Inkubationszeit die Verteilung des besagten Polypeptids beobachtet wird extrakorporal mit einer Gamma-Scintillations-Kamera oder einer magnetischen Resonanzmessung.

- Verfahren zur Feststellung des prothrombotischen Zustands, bei dem

    a) extrakorporal das zu untersuchende Blut mit einem antikoagulierenden Polypeptid oder Mittel aus der Familie der Annexine, vorzugsweise VAC das einen detektierbaren Marker trägt, vermischt wird und

    b) die mit spezifischen Zelltypen assoziierte Markierung analysiert wird.

Die Verwendung von

- Mittel, das neben einem mit einem detektierbaren Marker versehenem anti-koagulierendem Polypeptid aus der Familie der Annexine, vorzugsweise VAC zusätzlich einen Hilfsstoff beispielsweise physiologische Kochsalzlösung, TWEEN 80, Arginin und/oder Phosphatpuffer enthält, wobei gegebenenfalls ein nicht die Plasma-Calziumkonzentration reduzierendes Antikoagulant, vorzugsweise Heparin verwendet werden kann.

- Verwendung eines der erfindungsgemäßen antikoagulierenden Polypeptide oder Mittel zur Unterscheidung von Phosphatidylserin und Phosphatidylcholin.

4

- Verwendung eines der erfindungsgemäßen antikoagulierenden Polypeptide oder Mittel zur Diagnose des prothrombotischen Zustands oder des Ausgangspunktes der Störung des hämostatischen Systems oder des Thrombus.

Materialien und Methoden

VAC wurde entweder analog EPA 0 181 465 oder EPA 0 293 567 hergestellt. Die nachfolgenden Experimente wurden mit VACα durchgeführt, doch sind die Ergebnisse auch auf die anderen Annexine, insbesondere auch auf VACβ übertragbar.

Lipide

Dioleoyl-phosphatidylcholin (DOPC, Nr. P-1013) Dioleoyl-phosphatidylethanolamin (DOPE, Nr. P-0510), Cardiolipin (CL, Nr. C-5646), Dioleoyl-phosphatidylglycerol (DOPG, Nr. P-9664), Phosphatidylinositol (PI, Nr. P-0639), Dioleoyl-phosphatidsäure (DOPA, Nr. P-2767), Stearylamin (SA, S-6755) und Eigelb Sphingomyelin (S-0756) wurden von der Firma Sigma Chemical Co bezogen.

Die Reinheit von DOPC und DOPE wurde durch Dünnschichtchromatographie überprüft. Dioleoyl-phosphatidylserin (DOPS) wurde durch Konversion von DOPC gemäß (1) hergestellt. $^{14}$C markiertes DOPS (spez. Akt. 100.000 dpm/μg) wurde von Amersham bezogen.

Herstellung der Phospholipid-Doppelschichten auf Siliziumplatten.

Phospholipid Doppelschichten wurden mit einem "Langmuir-film balance" (Lauda Typ FW-1) wie bei Corsel et al. (2) beschrieben aufgetragen. Hydrophile Siliziumplatten wurden 24 Stunden in 30 % Chromschwefelsäure und Wasser behandelt und in 50 % Ethanol/Wasser aufbewahrt. Vor ihrer Verwendung wurden sie gründlich mit einem Detergenz und Wasser gereinigt.

Der "film balance" wurde mit demineralisiertem Wasser und 50 μM CaCl$_2$ gefüllt. Auf diese Unterphase wurden 20 μl einer Lösung, die ca. 2g/l Phospholipid in Chloroform enthält, aufgebracht. Die DOPS Fraktionen in den Doppelschichten wurden mit $^{14}$C-markiertem DOPS in Mischung mit DOPC überprüft. Die aufgebauten Doppelschichten wurden von den Siliziumplatten mit dem Szintillations-Detergenz (Du Pont Formel-989) entfernt und die Totalradioaktivität wurde in einem Szintillationszähler gemessen.

Messung der Bindung durch Ellipsometrie

Die Adsorption von VAC an die Phospholipid-Doppelschichten wurde mit Hilfe eines automatischen Ellipsometers wie beschrieben gemessen (2,3).

Die Bindungsversuche wurden in einer hydrophilen Küvette, die 5 ml eines gerührten Puffers enthielt (0,05 M Tris/HCl; 0,1 M NaCl; pH=7,5; T=20°C) durchgeführt. Die divalenten Kationen wurden als Chloride schrittweise zugegeben.

Bei VAC-Konzentrationen <0,1 μg/ml wurde der Puffer, der die spezifische VAC Konzentration enthielt, kontinuierlich zugegeben, um für VAC eine ausreichende Pufferkapazität zu schaffen.

Aus den kombinierten Polarisier- und Analysier-Daten wurde der refraktive Index und die Dicke d des adsorbierten Films bestimmt (4). Die Menge Γ der adsorbierten Proteinschicht wurde aus dem refraktiven Index und der Dicke mit Hilfe einer modifizierten Lorentz-Lorenz Gleichung [1] bestimmt (3,5):

$$\Gamma = 3d(n^2 - nb^2)/[(n^2 + 2)(r(nb^2 + 2) - v(nb^2 - 1))]; \qquad [1]$$

nb ist der refraktive Index des Puffers. Die Werte r=0,254 und v=0,71 wurden für die spezifische molare Refraktivität und des partiellen spezifischen Volumens eingesetzt (3).

Ergebnisse

Der Effekt von divalenten Kationen auf die Bindung des VAC an Phospholipide.

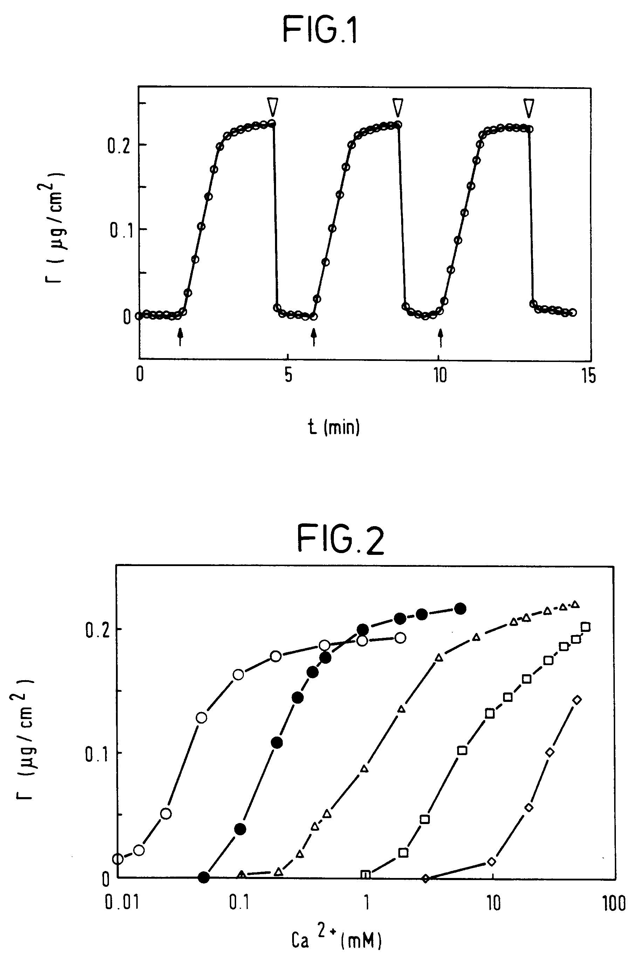

VAC bindet an Phospholipid-Membranen, die aus 20% DOPS/80% DOPC bestehen, in Abhängigkeit von der Calziumkonzentration. Anschließende Zugabe von EDTA führte zur sofortigen und vollständigen Desorption (Fig. 1). Durch die Veränderung der freien Ca$^{2+}$-Konzentration konnte die Adsorption mehrere Male wieder ausgelöst werden, ohne daß die adsorbierte Menge oder die Adsorptionsrate sich merklich veränderte. Irreversible Änderungen des VAC-Moleküls oder der Phospholipid-Doppelschichten durch die Adsorption oder Desorption sind daher nicht wahrscheinlich. Die Bindung war ebenfalls vollständig reversibel, wenn die Küvette mit $Ca^{2+}$-freiem Puffer gespült wurde.

Die $Ca^{2+}$-Abhängigkeit der VAC-Bindung an Phospholipide ist in Fig. 2 dargestellt. Die $Ca^{2+}$-Dosis-Wirkungs-Kurve zeigt ganz eindeutig eine $Ca^{2+}$-Konzentration, bei der die Hälfte der maximalen VAC-Adsorption erreicht wird: $[Ca^{2+}]_{1/2}$. Der $[Ca^{2+}]_{1/2}$-Wert hängt von der Zusammensetzung der Phospholipidoberfläche ab. Bei Phospholipidoberflächen, die 100%, 20%, 5% und 1% DOPS enthalten, wurden $[Ca^{2+})_{1/2}$-Werte von 36 µM, 220 µM, 1,5 mM bzw. 8,6 mM gemessen (Tab. 1). Diese Ergebnisse stimmen recht gut überein mit dem $[Ca^{2+}]_{1/2}$-Wert von 53 µM, der für die Endonexin II (=VAC)- Bindung an äquimolaren Mischungen von PS/PC Vesikeln gemessen wurde (6). Die maximale Menge des adsorbierten Proteins (Γmax) war unabhängig von der DOPS Fraktion der Membran und betrug ca. 0,217 µg/cm$^2$. Keine Adsorption konnte bei reinen DOPC-Doppelschichten bis zu einer $Ca^{2+}$ Konzentration von 3mM festge-stellt werden.

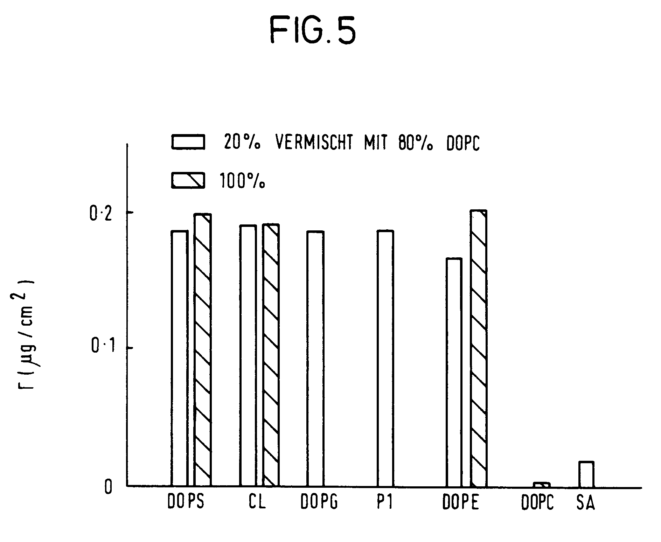

Die Adsorption von VAC an Phospholipid-Doppelschichten verschiedener Zusammensetzungen ist in Fig. 5 gezeigt. Auch hier wird deutlich, daß bei reinen DOPC-Doppelschichten praktisch keine Adsorption von VAC zu beobachten ist. Ebenfalls schwach ist die Adsorption des VAC an Stearylamid (SA).

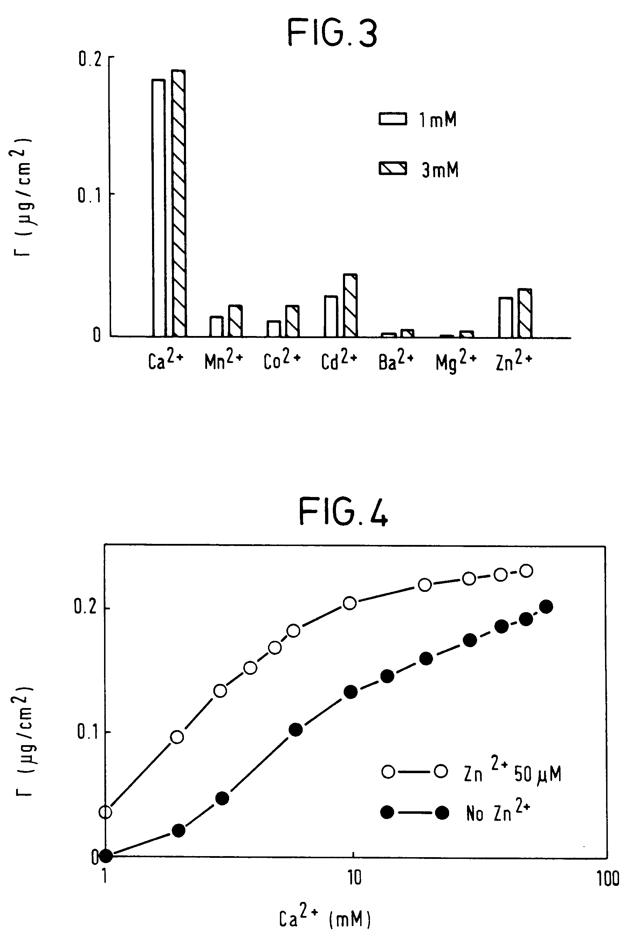

Bei Versuchen mit anderen Kationen als $Ca^{2+}$ wurde festgestellt, daß die Bindung von VAC an die Phospholipide in starkem Maße $Ca^{2+}$-spezifisch ist (Fig. 3). $Cd^{2+}$, $Zn^{2+}$, $Mn^{2+}$ und $Co^{2+}$ zeigten geringe Förderung der Bindung; $Ba^{2+}$ und $Mg^{2+}$ hatten keinen Einfluß. Diese Eigenschaft der Kationen kann in gewisser Weise mit ihren Ionenradien korre-liert werden.

<u>Zink Synergismus</u>

Hohe Konzentrationen an Zink-Ionen (1 mM) fördern nur in geringem Maß VAC-Adsorption (Fig. 3); 50 µM haben überhaupt keinen Einfluß auf die Adsorption. Überraschenderweise beeinflußt diese Konzentration die Bindung in Anwesenheit von $Ca^{2+}$; ein Synergismus ist zu beobachten. Der $[Ca^{2+}]_{1/2}$-Wert fiel von 8,6 auf 2,7 mM für Doppelschichten mit nur 1% DOPS ($[Zn^{2+}]$=50 µM.) (Fig. 4). 50 µM $[Zn^{2+}]$ liegt im normalen Bereich der Plasma Zink-Konzen-trationen.

Diagnostische Verfahren

1. <u>in vitro</u> Diagnose

a) VAC wird nach an sich bekannten Verfahren mit der Fluoresceingruppe, beispielsweise mit Hilfe von Fluoreszeinisothiocyanat markiert (13); man erhält auf diese Weise VAC-FITC.

b) Blut eines Patienten wird in einem Plastikröhrchen gesammelt, das ein nicht die Plasma-Calziumkonzentration reduzierendes Antikoagulant (z.B. Heparin) und VAC-FITC enthält.

c) Nach der Durchmischung werden die Blutzellen unter Verwendung eines FACS (fluorescence activated cell sorter) analysiert. Diese Analyse bestimmt die Fluoreszenzintensität, die mit spezifischen Zelltypen assoziiert ist.

d) Die Analysenprofile zeigen die Menge an Plättchen mit gebundenem VAC-FITC, d.h. Plättchen mit exponiertem PS, und daher das Vorliegen eines prothrombotischen Zustands an. Dieser gesundheitsgefährdende Zustand kann auf diese Art frühzeitig erkannt und folglich frühzeitig therapiert werden.

2. <u>in vivo</u> Diagnose

a) VAC wird mit einem kurzlebigen Isotop, beispielsweise $^{131}$I nach an sich bekannten Verfahren markiert; man erhält $^{131}$I-VAC.

b) $^{131}$I-VAC wird intravenös einem Patienten appliziert.

c) Nach einer gewissen Inkubationszeit wird der Patient einer Ganz- oder Teilkörperscintigraphie ausgesetzt. Die Verteilung der Radioaktivität kann mit einer large-field-of-view Gamma-Kamera beobachtet werden.

d) Intravaskuläre Stellen mit $^{131}$I-VAC Akkumulation kennzeichnen die Stelle, an der die Thrombose fortschreitet. Geeignete, thromboseverhindernde bzw. thromboseheilende Maßnahmen können frühzeitig ergriffen werden.

## 1. Radioaktive Markierung

### 1.1 Vorbereitungen

#### 1.1.1. Herstellung eines 500 mmol/l Natriumphosphatpuffers

24,5 g Natriinm-di-hydrogenphosphatmonohydrat wurden in 1 l bidest. Wasser gelöst und zu einer Lösung von 35,5 g Di-natrium-hydrogenphosphat in 1 l bidest. Wasser bis zum pH 7,5 zugesetzt.

#### 1.1.2. Herstellung eines 20 mmol/L Natriumphosphatpuffers + 150 mmol NaCl (Elutionspuffer)

2,76g Natrium-di-hydrogenphosphatmonohydrat wurden in 1 l bidest. Wasser gelöst und zu einer Lösung von 2,84 g Di-natrium-hydrogenphosphat in 1 l bidest. Wasser bis zum pH 7,2 zugesetzt. Durch Zugabe von 8,77 g NaCl (150 mmol) zu 1 l des Puffers wurde der Elutionspuffer hergestellt.

#### 1.1.3. Equilibieren der Reinigungssäule

Eine PD-10-Säule (Sephadex G25, Fa. Pharmacia) wurde mit ca. 30 ml des Elutionspuffers equilibriert.

#### 1.1.4. Vorbereitung des Reaktionsgefäßes

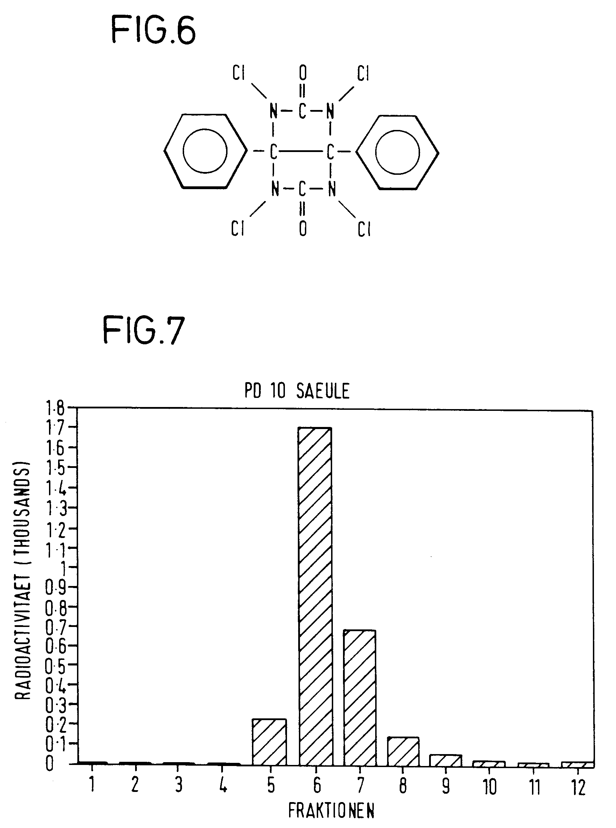

2 mg IODO-Gen (Molmasse: 432,09 g/mol; Fig. 6) wurden in 50 ml Dichlormethan, reinst, gelöst. 200 $\mu$l dieser Lösung wurden in ein 1,5 ml Eppendorf-Gefäß pipettiert und dann bei 37°C (Thermostatblock) das Lösungsmittel abgedampft. Im Reaktionsgefäß waren damit 8 $\mu$g ($1,85 \times 10^{-2}$ mmol) IODO-Gen feinst verteilt von der Wand aufgebracht.

#### 1.1.5. Zur Markierung eingesetztes VAC-$\alpha$

Ausgegangen wurde von der Lösung von 50 mg VAC-$\alpha$ in 4 ml 20 mmol/l Natriumphosphatpuffer + 150 mmol/l NaCl, pH 7,2, verdünnt mit 1 ml bidest. Wasser. Molmasse VAC-$\alpha$: 34000 g/mol.

#### 1.1.6. Zur Markierung eingesetztes J-125

Na$^{125}$J von der Fa. Dupont, NEN Products, mit 67,3 MBq (=1,82 mCi) Gesamtradioaktivität am Kalibrierungstag. Die spezifische Aktivität war 15,9 Ci/mg Jod = 1,98 kCi/mmol 1/2 $J_2$, das entsprach 0,115 $\mu$g Jod ($9,2 \times 10^{-7}$ mmol 1/2 $J_2$). Das aktive NaJ war in 5,5 $\mu$l 0,1 mol/l NaOH gelöst.

### 1.2. Jodierung

Alle Arbeiten wurden in Isotopenabzug hinter Bleiglasabschirmungen durchgeführt.
20 $\mu$l (= 200 $\mu$g) der unter 2.1.5 beschriebenen Lösung von VAC-$\alpha$ wurden in das IODO-Gen vorbehandelte Reaktionsgefäß überführt. Dieses wurde verschlossen und 20 Minuten bei Raumtemperatur geschüttelt. Danach wurde die Reaktionslösung mit einer Pipette auf die vorbereitete PD-10-Säule (s. 2.1.3.) aufgebracht. Das Reaktionsgefäß wurde noch mit 500 $\mu$l des Elutionspuffers (s. 2.1.2.) nachgespült und diese Lösung ebenfalls auf die PD-10-Säule gebracht. Das dabei abfließende Eluat wurde verworfen.

### 1.3. Reinigung

Durch Aufbringen von jeweils 0,5 ml Elutionspuffer (s. 2.1.2.) in 2-Minuten-Abständen, wurde nun das VAC-$\alpha$ [$^{125}$J] von dem noch freien $^{125}$J/Na $^{125}$J abgetrennt. Nach 12 Fraktionen war dieser Reinigungsschritt beendet. Der relative Aktivitätsgehalt der Fraktion wurde mit einem Labormonitor (GMZ) gemessen (Abb. 2).
Die Fraktionen 6 und 7 wurden vereinigt, mit Elutionspuffer auf genau 2,0 ml aufgefüllt, in 100 $\mu$l-Portionen aufgeteilt und bei -20°C eingefroren. Die Substanz stand in diesen Portionen für Analytik und Entwicklungsarbeiten zur Verfügung.

2. Analytischer Teil

## 2.1 Gehaltsbestimmung

Im chromogenen Substratassay wurde ein VAC-$\alpha$-Gehalt von 71,8 $\mu$g/2,0 ml Lösung gemessen.

## 2.2 Radioaktivitätsbestimmung

### 2.2.1. Gesamtradioaktivität

Nach dem Auftauen einer 100 $\mu$l-Portion wurden 50 $\mu$l dieser [125] VAC-$\alpha$-Lösung zu 950 $\mu$l der inaktiven VAC-$\alpha$-Lösung (s. 2.1.5.) hinzupipettiert, gut vermischt und davon jeweils 50 $\mu$l zur Messung im LSC-Counter (Beckman) gebracht. 24,5 MBq (= 0,663 mCi) / 2,0 ml Gesamtlösung.

### 2.2.2. Spezifische Aktivität

Aus Gehaltsbestimmung und Gesamtradioaktivität ergab sich eine spezifische Aktivität von 341,5 MBq/mg = 11,61 TBq/mmol (9,23 mCi/mg = 313,8 Ci(mmol)

### 2.2.3. Bestimmung der proteingebundenen Radioaktivität durch TCA-Fällung

100 $\mu$l der VAC-$\alpha$-Lösung wurden mit 50 $\mu$l 3% iger BSA-Lösung und 150 $\mu$l 40 %iger wäßriger Trichloressigsäure versetzt, gut durchgeschüttelt und 60 Minuten im Kühlschrank stehengelassen. Der entstandene Niederschlag wurde abzentrifugiert. Aliquote Mengen des Überstandes wurden im LSC-Counter zur Messung gebracht.

Ergebnis: 99,3% der Radioaktivität waren fällbar.

### 2.2.4. Radioaktivitätsausbeute

Von den eingesetzten 67,3 MBq wurden 24,5 MBq im VAC-$\alpha$ wiedergefunden. Somit betrug die Aktivitätsausbeute 36,4 %.

## 2.3 Modifikationsgrad

Aus der spezifischen Aktivität und der eingesetzten Menge an inaktivem VAC-$\alpha$ wurde errechnet, daß statistisch jedes 6. VAC-$\alpha$ Molekül mit einem $^{125}$J-Atom markiert wurde.

## 3.4 Identität und Reinheit

Unter Anwendung der SDS-PAGE (Gradientengel 7 - 17 %, nicht reduzierende Bedingungen) und der anschließenden Auswertung des Geles durch Silberfärbung (Oakley-Methode), Autoradiographie und Detektion unter dem Linearanalyzer (Fa. Berthold LB 282, Sonde LB 2820) (Abb. 3) wurde die Substanz im Vergleich zum eingesetzten VAC-$\alpha$ untersucht. Die Substanzen waren identisch, der Anteil an dimerem Produkt lag deutlich unter der für inaktive Chargen tolerierten Grenze von 8%.

Legende zu den Figuren

Fig. 1: Alternierende Adsorption und Desorption von VAC an eine Phospholipidoberfläche, induziert durch Erhöhung bzw. Erniedrigung der $Ca^{2+}$-Konzentration. Die Adsorption von VAC (1 $\mu$g/ml) an eine 20 % DOPS/80 % DOPC Phospholipid-Doppelschicht. Zugabe, von $Ca^{2+}$ (3,4,6 mM) ist durch ↑ bzw. ▽ gekennzeichnet.

Fig. 2: Einfluß der Phospholipid-Zusammensetzung und der $Ca^{2+}$ Konzentration auf die Adsorption von VAC auf eine Phospholipidoberfläche. o 100 % DOPS; o 20 % DOPS; Δ 5 % DOPS; □ 1 % DOPS; 100 % DOPC; sämtliche Mischungen wurden ergänzt mit DOPC. [VAC] = 1 $\mu$g/ml.

Fig. 3: Effekt von bivalenten Ionen auf die Adsorption von VAC. VAC Adsorption an Doppelschichten aus 20 % DOPS und 80 % DOPC in Gegenwart der angegebenen Ionen (1 oder 3 mM). [VAC] = 1 $\mu$g/ml.

Fig. 4: Synergistischer Effekt von $Zn^{2+}$ auf die $Ca^{2+}$-abhängige Adsorption von VAC an die Phospholipidoberfläche. Der Effekt von $Ca^{2+}$ auf die VAC Adsorption an 1 % DOPS und 99 % DOPC in Gegenwart von 50 μM $Zn^{2+}$ wurde gemessen. [VAC] = 1 μg/ml.

Fig. 5: Adsorption von VAC an Phospholipid-Doppelschichten verschiedener Zusammensetzungen. VAC Adsorption an Dioleoylphosphatidylserin (DOPS), Cardiolipin (CL) und Dioleoylphosphatidylethanolamin (DOPE) entweder rein oder vermischt mit 80 % Dioleoylphosphatidylcholin (DOPC), an Dioleoylphosphatidylglycerol (DOPG), Phosphatidylinositol (PI) und Stearylamin vermischt mit 80 % DOPC oder an reines DOPC. [VAC] = 1 μg/ml; $[Ca^{2+}]$ = 3 mM.

Fig. 6:
1,3,4,6-Tetrachloro-3α-6α-diphenyl-glycoluril (IODO-GEN)

Fig. 7: Radioaktivitätsverteilung in den Fraktionen 1-12.

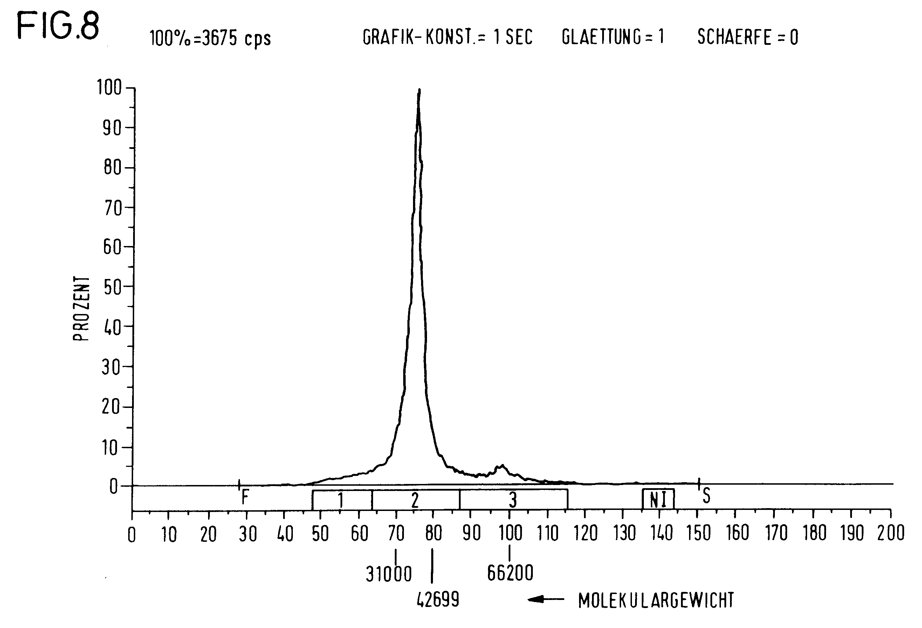

Fig. 8: Radioaktivitätsverteilung unter dem Linearanalyzer

Tabelle 1

| Evaluierung der Phospholipid-Doppelschichten, die auf Siliziumplatten aufgetragen waren. | | | |
|---|---|---|---|
| $^{14}$C-DOPS auf film balance % | Menge an Phospholipiden (ellipsometrisch) μg/cm$^2$ | Aktivität DPM | DOPS Fraktion gemessen % |
| 2 | 0,396 | 453 | 1,9 |
| 5 | 0,409 | 1133 | 4,5 |
| 20 | 0,401 | 5006 | 20 |
| 100 | 0,442 | 27174 | 99 |

Die kalkulierte Mischung wurde auf dem "film balance" aufgetragen. Die Menge der Doppelschicht wurde durch Ellipsometrie gemessen und die Aktivität des $^{14}$C-markierten DOPS wurde mit einem Beckmann 6S 3801 Szintillationszähler (s.d. <2%) gemessen und um die Hintergrundstrahlung korrigiert (60 DPM). Die DOPS-Fraktion in der Doppelschicht wurde durch Gleichung 2 berechnet:

$$\text{Fraktion} = \frac{\text{Menge}(\mu g/cm^2) \text{ x spez. Aktivität (DPM.}\mu g^{-1})}{\text{Aktivität (DPM) x Fläche (cm}^2)}$$

Die spezifische Aktivität von DOPS betrug 100.000 DPM.μg$^{-1}$, die mit Phospholipiden besetzte Oberfläche 0,62 cm$^2$.

Tabelle 2

| Die Hälfte der maximalen VAC-Bindung an verschiedene Phospholipidoberflächen. | | |
| --- | --- | --- |
| Lipid (mol%/mol%) | $\Gamma$max ± S.D. ($\mu$g/cm$^2$) | $[Ca^{2+}]_{1/2}$±S.D. mM |
| DOPS (100) | 0,195 ± 0,025 | 0,036 ± 0,013 |
| DOPS / DOPC (20/80) | 0,222 ± 0,014 | 0,22 ± 0,06 |
| DOPS / DOPC (5/95) | 0,229 ± 0,004 | 1,5 ± 0,5 |
| DOPS / DOPC (1/99) | 0,234 ± 0,007 | 8,6 ± 2,5 |
| Cardiolipin / DOPC (20/80) | 0,209 ± 0,011 | 0,039 ± 0,022 |
| DOPG / DOPC (20/80) | 0,212 ± 0,003 | 0,155 ± 0,027 |
| PI / DOPC (20/80) | 0,221 ± 0,005 | 0,47 ± 0,05 |
| DOPA / DOPC (20/80) | 0,207 ± 0,006 | 0,75 ± 0,26 |
| DOPE / DOPC (20/80) | 0,213 ± 0,003 | 0,86 ± 0,21 |
| Sphingomyelin / DOPC (20/80) | 0,225 ± 0,014 | 7 ± 3 |
| DOPC (100) | n.d. | >30 mM |

Die maximale VAC-Adsorption ($\Gamma$max) an die aufgeführten Phospholipidoberflächen zusammen mit der Calzium-Konzentration, die zur Hälfte der maximalen VAC-Bindung $[Ca^{2+}]_{1/2}$ führt, sind als Mittelwerte aus wenigstens drei verschiedenen Experimenten mit den entsprechenden Standard-Abweichungen angegeben.
n.d. = not determined = nicht bestimmt.

### Literaturverzeichnis

1. Confurius, P & Zwaal, R. F. A. (1977) Biochim. Biophys. Acta 488, -42.

2. Corsel, J. W., Willems, G. M., Kop, J. M. M., Cuypers, P. A. & Hermens, W. Th. (1986) J. Colloid Interface Sci. 111, 544-554.

3. Cuypers, P. A., Corsel, J. W., Janssen, M. P., Kop, J. M. M., Hermens, W. TH. & Hemker, H. C. (1983) J. Biol. Chem. 258, 2426-2431.

4. McCrackin, F. L., Passaglia, E., Stromberg, R. R. & Steinberg, H. L. (1963) J.Res.Nat.Bur.Stand.Sect.A 67, 3-377.

5. Kop, J. M. M., Cuypers, P. A., Lindhout, Th., Hemker, H. C. & Hermens, W. Th. (1984) J. Biol. Chem. 259, 13993-13998.

6. Schlaepfer, D. D., Mehlman, T., Burgess, W. H. & Haigler. H. T. (1987) Proc. Natl. Acad. Sci. USA 84, 6078-6082.

7. Op den Kamp, J.A. F. Ann. Rev. Biochem. 1979, 48: 47-71.

8. Zwaal, R.F.A. Biochim. Biophys. Acta 1978, 515: 163-205.

9. Jackson, C.M. und Nemerson, Y. Ann. Rev. Biochem. 1980, 49: 765-811.

10. Bevers, E.M., Comfurius, P. und Zwaal, R.F.A. Biochim. Biophys. Acta 1983, 736: 57-66.

11. Rosing, J., Bevers, E.M., Comfurius, P., Hemker, H.C. can Dieijen, G., Weiss, H.J. und Zwaal, R.F.A. Blood 1985, 65: 1557-1561.

12. Fraker P.J., Speck, J.C., Biochem. Biophys. Res. Comm. 80, 849-857, 1978.

EP 0 509 026 B1

13. Reisher, J.I. & Orr, H.C., Anal. Biochem. 1968, 26, 178-179.

**Patentansprüche**

1. Verwendung eines antikoagulierenden Polypeptides aus der Familie der Annexine, dadurch gekennzeichnet, daß es mit einem detektierbaren Marker versehen wird, als Mittel zur Unterseheidung zwischen Phosphatidylcholin und Phosphatidylserin.

2. Verwendung eines antikoagulierenden Polypeptids aus der Familie der Annexine, dadurch gekennzeichnet, daß es mit einem detektierbaren Marker versehen wird, als Mittel zur Diagnose des prothrombischen Zustands, des Ausgangspunkts der Störung oder Aktivierung des hämostatisehen Systems und/oder Thrombus.

3. Verwendung eines Polypeptids gemäß Anspruch 1 oder 2 dadurch gekennzeichnet, daß es sich um VAC handelt.

4. Verwendung eines Polypeptids gemäß einem der Ansprüche 1 bis 3, dadurch gekennzeichnet, daß es sich um VAC-$\alpha$ oder VAC-$\beta$ handelt.

5. Verwendung eines Polypeptids gemäß einem der Ansprüche 1 bis 4, dadurch gekennzeichnet, daß zur Fluoreszenzmarkierung vorzugsweise Fluoresceinisothiocyanat, oder zur radioaktiven Markierung ein Radioisotop eines Halogens, des Technetiums, Bleis, Quecksilbers, Thalliums oder des Indiums, besonders bevorzugt $^{131}$I oder $^{125}$I oder daß ein paramagnetisches Kontrastagenz eingesetzt wird.

6. Verwendung eines Polypeptids gemäß einem der Ansprüche 1 bis 5, dadurch gekennzeichnet, daß das Mittel zusätzlich einen Hilfsstoff enthält.

7. Verwendung eines Polypeptids gemäß Anspruch 6, dadurch gekennzeichnet, daß als Hilfsstoff physiologische Kochsalzlösung, Arginin und/oder Phosphatpuffer verwendet wird.

8. Verwendung eines Polypeptids gemäß einem der Ansprüche 1 bis 7, dadurch gekennzeichnet, daß das Mittel zusätzlich ein nicht die Plasma-Calciumkonzentration reduzierendes Antikoagulant, vorzugsweise Heparin, enthält.

9. Verfahren zur Feststellung des Ausgangspunktes für die Aktivierung des hämostatischen Systems, dadurch gekennzeichnet, daß

   a) ein mit einem detektierbaren Marker versehenes antikoagulierendes Polypeptid aus der Familie der Annexine in das System eingebracht wird und

   b) nach einer Inkubationszeit die Verteilung des besagten Polypeptids beobachtet wird.

10. Verfahren gemäß Anspruch 9, dadurch gekennzeichnet, daß das Polypeptid VAC ist.

11. Verfahren gemäß Anspruch 9 oder 10, dadurch gekennzeichnet, daß das Polypeptid VAC-$\alpha$ oder VAC-$\beta$ ist.

12. Verfahren gemäß einem der Ansprüche 9 bis 11, dadurch gekennzeichnet, daß als detektierbarer Marker ein Radioisotop, vorzugsweise eines der Radioisotope $^{125}$I, $^{123}$I, $^{131}$I, $^{111}$In, $^{99m}$Tc, $^{203}$Pb, $^{198}$Hg oder $^{201}$Tl oder ein paramagnetisches Kontrastelement verwendet wird.

13. Verfahren gemäß einem der Ansprüche 9 bis 12, dadurch gekennzeichnet, daß das Polypeptid intraarteriell oder intravenös verabreicht wird.

14. Verfahren gemäß einem der Ansprüche 9 bis 13, dadurch gekennzeichnet, daß die Verteilung des besagten Polypeptids extrakorporal mit einer Gamma-Scintillations-Kamera oder einer magnetischen Resonanzmessung beobachtet wird.

15. Verfahren zur Feststellung des prothrombotischen Zustands, dadurch gekennzeichnet, daß

   a) extrakorporal das zu untersuchende Blut mit einem antikoaginlierenden Polypeptid aus der Familie der Annexine, das einen detektierbaren Marker trägt, vermischt wird und

b) die mit spezifischen Zelltypen assoziierte Markierung analysiert wird.

16. Verfahren gemäß Anspruch 15, dadurch gekennzeichnet, daß das Polypeptid VAC ist.

17. Verfahren gemäß Anspruch 15 oder 16, dadurch gekennzeichnet, daß als detektierbarer Marker die Fluoresceingruppe oder ein Radioisotop, vorzugsweise eines der Radioisotope $^{125}$I, $^{123}$I, $^{131}$I, $^{111}$In, $^{99m}$Tc, $^{203}$Pb, $^{198}$Hg oder $^{201}$Tl verwendet wird.

18. Verfahren gemäß einem der Ansprüche 9 bis 17, dadurch gekennzeichnet, daß zusätzlich ein Hilfsstoff zugesetzt wird.

19. Verfahren gemäß Anspruch 18, dadurch gekennzeichnet, daß als Hilfsstoff physiologische Kochsalzlösung, Arginin und/oder Phosphatpuffer verwendet wird.

20. Verfahren gemäß Anspruch 18 oder 19, dadurch gekennzeichnet, daß zusätzlich ein nicht die Plasma-Calziumkonzentration reduzierendes Antikoagulant, vorzugsweise Heparin, verwendet wird.

## Claims

1. Use of an anticoagulant polypeptide from the family of annexines, characterised in that it is provided with a detectable marker, as a means of distinguishing between phosphatidyl choline and phosphatidyl serine.

2. Use of an anticoagulant polypeptide from the family of annexines, characterised in that it is provided with a detectable marker, as a means of diagnosing the prothrombotic state, the starting point of a disorder or activation of the haemostatic system and/or thrombus.

3. Use of a polypeptide according to claim 1 or 2, characterised in that it is VAC.

4. Use of a polypeptide according to one of claims 1 to 3, characterised in that it is VAC-$\alpha$ or VAC$\beta$.

5. Use of a polypeptide according to one of claims 1 to 4, characterised in that, for fluorescent labelling, fluorescein isothiocyanate is preferably used, or, for radioactive labelling, a radioisotope of a halogen, technetium, lead, mercury, thallium or indium, particularly preferably $^{131}$I or $^{125}$I, is used, or in that a paramagnetic contrast agent is used.

6. Use of a polypeptide according to one of claims 1 to 5, characterised in that the agent additionally contains an adjuvant.

7. Use of a polypeptide according to claim 6, characterised in that physiological saline solution, arginine and/or phosphate buffer is used as the adjuvant.

8. Use of a polypeptide according to one of claims 1 to 7, characterised in that the agent additionally contains an anticoagulant, preferably heparin, which does not reduce the plasma calcium concentration.

9. Process for detecting the starting point of activation of the haemostatic system, characterised in that

a) an anticoagulant polypeptide, provided with a detectable marker, from the family of annexines is introduced into the system and

b) the distribution of said polypeptide is observed after an incubation period.

10. Process according to claim 9, characterised in that the polypeptide is VAC.

11. Process according to claims 9 or 10, characterised in that the polypeptide is VAC-$\alpha$ or VAC-$\beta$.

12. Process according to one of claims 9 to 11, characterised in that a radioisotope, preferably one of the radioisotopes $^{125}$I, $^{123}$I, $^{131}$I, $^{111}$In, $^{99m}$Tc, $^{203}$Pb, $^{198}$Hg or $^{201}$Tl, or a paramagnetic contrast element is used as the detectable marker.

13. Process according to one of claims 9 to 12, characterised in that the polypeptide is administered by intraarterial or intravenous route.

14. Process according to one of claims 9 to 13, characterised in that the distribution of said polypeptide is observed extracorporally, using a gamma scintillation camera or magnetic resonance measurement.

15. Process for detecting the prothrombotic state, characterised in that

 a) the blood to be examined is mixed extracorporally with an anticoagulant polypeptide from the family of annexines which carries a detectable marker and

 b) the labelling associated with specific types of cells is analysed.

16. Process according to claim 15, characterised in that the polypeptide is VAC.

17. Process according to claim 15 or 16, characterised in that the fluorescein group or a radioisotope, preferably one of the radioisotopes $^{125}$I, $^{123}$I, $^{131}$I, $^{111}$In, $^{99m}$Tc, $^{203}$Pb, $^{198}$Hg or $^{201}$Tl, is used as the detectable marker.

18. Process according to one of claims 9 to 17, characterised in that an adjuvant is additionally used.

19. Process according to claim 18, characterised in that physiological saline solution, arginine and/or phosphate buffer is used as the adjuvant.

20. Process according to claim 18 or 19, characterised in that an anticoagulant, preferably heparin, which does not reduce the plasma calcium concentration is additionally used.

**Revendications**

1. Utilisation d'un polypeptide anticoagulant de la famille des annexines caractérisée en ce qu'il est muni d'un marqueur détectable, comme agent pour la discrimination entre la phosphatidylcholine et la phosphatidylsérine.

2. Utilisation d'un polypeptide anticoagulant de la famille des annexines caractérisée en ce qu'il est muni d'un marqueur détectable, comme agent pour le diagnostic de l'état prothrombotiqne, du point de départ de la perturbation ou de l'activation du système hémostatique et/ou d'un thrombus.

3. Utilisation d'un polypeptide selon la revendication 1 ou 2 caractérisée en ce qu'il s'agit de VAC.

4. Utilisation d'un polypeptide selon l'une des revendications 1 à 3 caractérisée en ce qu'il s'agit de VAC-α ou de VAC-β.

5. Utilisation d'un polypeptide selon l'une des revendications 1 à 4 caractérisée en ce que l'on utilise pour le marquage par fluorescence de préférence l'isothiocyanate de fluorescéine, ou pour le marquage radioactif un radio-isotope d'un halogène, du technétium, du plomb, du mercure, du thallium ou de l'indium, de manière particulièrement préférée $^{131}$I ou $^{125}$I, ou en ce que l'on utilise un agent de contraste paramagnétique.

6. Utilisation d'un polypeptide selon l'une des revendications 1 à 5 caractérisée en ce que l'agent contient en outre une substance auxiliaire.

7. Utilisation d'un polypeptide selon la revendication 6 caractérisée en ce que l'on utilise comme substance auxiliaire du sérum physiologique, l'arginine et/ou un tampon phosphate.

8. Utilisation d'un polypeptide selon l'une des revendications 1 à 7 caractérisée en ce que l'agent contient en outre un anticoagulant qui ne réduit pas la concentration plasmatique du calcium, de préférence l'héparine.

9. Procédé de détermination du point de départ de l'activation du système hémostatique caractérisé en ce que

a) un polypeptide anticoagulant de la famille des annexines muni d'un marqueur détectable est introduit dans le système et

b) après une période d'incubation la répartition dudit polypeptide est observée.

10. Procédé selon la revendication 9 caractérisé en ce que le polypeptide est VAC.

11. Procédé selon la revendication 9 ou 10 caractérisé en ce que le polypeptide est VAC-$\alpha$ ou VAC-$\beta$.

12. Procédé selon l'une des revendications 9 à 11 caractérisé en ce que l'on utilise comme marqueur détectable un radio-isotope, de préférence l'un des radio-isotopes $^{125}$I, $^{123}$I, $^{131}$I, $^{111}$In, $^{99m}$Tc, $^{203}$Pb, $^{198}$Hg ou $^{201}$Tl ou un élément de contraste paramagnétique.

13. Procédé selon l'une des revendications 9 à 12 caractérisé en ce que le polypeptide est administré par voie intra-artérielle ou intraveineuse.

14. Procédé selon l'une des revendications 9 à 13 caractérisé en ce que la répartition dudit polypeptide est observée extracorporellement avec une caméra à scintillation à rayons gamma ou une mesure de résonance magnétique.

15. Procédé pour la mise en évidence de l'état prothrombotique caractérisé en ce que

a) le sang à étudier est mélangé extracorporellement avec un polypeptide anticoagulant de la famille des annexines qui porte un marqueur détectable et

b) le marquage associé à des types cellulaires spécifiques est analysé.

16. Procédé selon la revendication 15 caractérisé en ce que le polypeptide est VAC.

17. Procédé selon la revendication 15 ou 16 caractérisé en ce que l'on utilise comme marqueur détectable le groupe fluorescéine ou un radio-isotope, de préférence l'un des radio-isotopes $^{125}$I, $^{123}$I, $^{131}$I, $^{111}$In, $^{99m}$Tc, $^{203}$Pb, $^{198}$Hg ou $^{201}$Tl.

18. Procédé selon l'une des revendications 9 à 17 caractérisé en ce que l'on ajoute en outre une substance auxiliaire.

19. Procédé selon la revendication 18 caractérisé en ce que l'on utilise comme substance auxiliaire du sérum physiologique, l'arginine et/ou un tampon phosphate.

20. Procédé selon la revendication 18 ou 19 caractérisé en ce que l'on utilise en outre un anticoagulant qui ne réduit pas la concentration plasmatique du calcium, de préférence l'héparine.

# FIG.1

# FIG.2

# FIG.3

# FIG.4

# FIG.5

# FIG.6

# FIG.7

PD 10 SAEULE

RADIOACTIVITAET (THOUSANDS)

FRAKTIONEN

FIG.8    100%=3675 cps        GRAFIK- KONST.= 1 SEC    GLAETTUNG = 1    SCHAERFE = 0

EP 0 509 026 B1